Europäisches Patentamt

(19) European Patent Office

Office européen des brevets

(11) Publication number: **0 232 845**
**A2**

(12) **EUROPEAN PATENT APPLICATION**

(21) Application number: **87101490.8**

(22) Date of filing: **04.02.87**

(51) Int. Cl.⁴: **C 12 N 15/00,** C 12 N 5/00

(30) Priority: **06.02.86 US 826679**

(43) Date of publication of application: **19.08.87**
**Bulletin 87/34**

(84) Designated Contracting States: **AT BE CH DE ES FR GB**
**GR IT LI LU NL SE**

(71) Applicant: **THE GENERAL HOSPITAL CORPORATION,**
**55 Fruit Street, Boston MA 02114 (US)**

(72) Inventor: **Kingston, Robert E., 21 Union Street, Brighton**
**Massachusetts 02135 (US)**
Inventor: **Wurm, Florian M., 744 Edgewater Boulevard**
**Apartment No. 210, Foster City California 94404 (US)**

(74) Representative: **Meyer-Dulheuer, Karl-Hermann, Dr. et**
**al, HOECHST Aktiengesellschaft Zentrale**
**Patentabteilung Postfach 80 03 20,**
**D-6230 Frankfurt/Main 80 (DE)**

(54) **Inducible heat shock and amplification system.**

㋨ The invention is directed to a method for high level polypeptide or protein production in transformed host cells. The
method comprises transforming a host cell with a structural
gene, encoding for the desired polypeptide or protein, operably
linked to an inducible heat shock promoter. After the copy
number of the structural gene is increased substantially
through gene amplification, the inducible heat shock promoter
is then induced to produce the polypeptide or protein.

## INDUCIBLE HEAT SHOCK AND AMPLIFICATION SYSTEM

Field of the Invention

This invention is in the field of genetic engineering and is directed to a method for high level polypeptide or protein production in transformed host cells.

Background of the Invention

Genetic engineering can be used to express or produce heterologous (foreign) polypeptides or proteins in transformed host cells. By these methods, a heterologous gene coding for the desired polypeptide or protein is inserted into an expression vector which is then introduced into the host cells. The replication system of the transformed host cells then reproduces the inserted gene. Under proper conditions, the replicated gene is expressed, producing both the host cell protein and the heterologous polypeptide or protein. The heterologous polypeptide or protein yield depends on a number of factors, including the copy number of the heterologous gene, the efficiency of the replication system of the host cell, and the type of promoters operably linked to the heterologous gene which encodes for the desired, polypeptide or protein.

Gene amplification can be used to increase the copy number of a plasmid or gene which a host cell contains. Although the use of a gene amplification system can result in high copy number of the desired heterologous gene, expression of the heterologous gene to produce the heterologous protein has not always proved successful.

In one study, Lau et al., Molecular and Cellular Biology, 4:1469-1475 (1984), the dihydrofolate reductase gene amplification system was used to increase the copy number of a heterologous human gene in Chinese hamster ovary cells. In this study, a mammalian inducible promoter, metallothionein, was used to control the expression of the human globin gene. After induction of the metallothionein promoter with cadmium, transcription of the gene occurred, but the amount of messenger RNA produced was insufficient to support translation into the globin protein.

In another study, inducible expression occurred, but at relatively low yields. Page, M., Gene, 37:139-144 (1985) describes the expression of human beta-interferon genes, amplified by the dihydrofolate reductase system, in Chinese hamster ovary cells. The beta-interferon gene was operably linked to the inducible promoter metallothionein, which was induced by cadmium sulfate. The results showed only a 1.2 to 10.7 increase in production of the beta-interferon than that produced without induction (Table I).

Many proteins of interest are synthesized by biological systems in minute quantities, which restricts progress toward understanding their function and medical application. Larger quantities of the protein would enable the purification and utilization of these proteins in therapeutic application. Genetic engineering cloning techniques are capable of isolating genes encoding these proteins. Further, genetic engineering techniques may be able to synthesize large quantities of these proteins in transformed host systems. For a number of reasons, including the glycosylation patterns and secondary structures of some of these proteins, synthesis in a mammalian host cell expression system is preferable.

It would be desirable to have a method for high level polypeptide or protein production, including heterologous polypeptides or proteins, in a gene amplification systen using an inducible promoter, such that after the gene amplification, the copy number of the heterologous gene in the host cell is significant, and upon induction, the inducible promoter retains a high level of inducibility, resulting in high level production of the desired polypeptide or protein.

Summary of the Invention

This invention provides for a method of high level polypeptide or protein production, including production of heterologous polypeptides and proteins, in a transformed host cell using a gene amplification system with an inducible heat shock promoter.

The invention was achieved in a method which comprises the steps of:

(a) transforming a host cell with a heterologous gene encoding for a polypeptide or protein, under the control of a heat shock promoter;

(b) amplifying the copy number of said heterologous gene in said transformed host cells using an amplification system under the control of a promoter other than an inducible heat shock promoter;

(c) inducing said inducible promoter by heat shocking said transformed host cells at a temperature and for a time sufficient to transcribe said heterologous gene; and

(d) allowing said heat shocked cells to recover at a lower temperature than said heat shock temperature and for a time sufficient to translate said transcribed heterologous gene, producing said heterologous polypeptide or protein.

This invention also provides for a method of high level production of a heterologous polypeptide or protein under the control of an inducible heat shock promoter in a transformed mammalian host cell, using the dihydrofolate reductase gene amplification system to substantially increase the copy number of the heterologous gene.

Description of the Figures

Figure 1a shows schematically the genetic map of the pCVSVE 11-DHFR plasmid. Figure 1b shows schematically the genetic map of the pHS-CMYC plasmid.

Figure 2 shows the copy number of c-myc constructs in recombinant cell lines.

Figures 3A and 3B show the presence, inducibility, and stability of c-myc mRNA in recombinant CHO cells. Figure 3C shows a comparison of c-myc mRNA sequences in Balb c/3T3, CHO-DUKX Bl cells and cells from recombinant CHO cell lines.

Figures 4A and 4B show the time course induction of c-myc protein and analysis of protein levels in induced cells. Figure 4C shows the radiolabelling of cells after heat shock and the immunoprecipitation of the myc protein.

Definitions

In the description that follows, a number of terms used in recombinant DNA technology are extensively utilized. In order to provide a clearer and consistent understanding of the specification and claims, including the scope to be given such terms, the following definitions are provided.

Promoter. A DNA sequence generally described as the 5' region of gene, located proximal to the start codon. At the promoter region, transcription or expression of an adjacent gene(s) is initiated.

Polynucleotide molecule. A linear sequence of nucleotides linked together by a backbone consisting of an alternating series of sugar and phosphate residues and as used herein can include DNA and RNA polymers.

Gene. A DNA sequence that contains information for construction of a polypeptide or protein, and as used herein, includes the 5' and 3' ends.

Structural gene. A DNA sequence that is transcribed into messenger RNA that is then translated into a sequence of amino acids characteristics of a specific polypeptide. Typically the first nucleotide of the first translated codon is numbered +1, and the nucleotides are numbered consecutively with positive integers through the translated region of the structural gene and into the 3' untranslated region. The numbering of nucleotides in the promoter and regulatory region 5' to the translated region proceeds consecutively with negative integers with the 5' nucleotide next to the first translated nucleotide being numbered -1.

Heterologous gene. A structural gene that is foreign, i.e. originating from a donor different from the host or chemically synthesized gene, and can include a donor of a different species from the host. The gene codes for polypeptide ordinarily not produced by the organism susceptible to transformation by the expression vehicle.

Operably linked. As used herein means that the promoter controls the initiation of the expression of the polypeptide encoded by the structural gene.

Expression. Expression is the process by which a structural gene produces a polypeptide. It involves transcription of the gene into messenger RNA (mRNA) and the translation of such mRNA into polypeptide(s).

Cloning vehicle. A plasmid or phage DNA or other DNA sequence which is able to replicate in a host cell, which is characterized by one or a limited number of endonuclease recognition sites at which such DNA sequence may be cut in a determinable fashion without loss of an essential biological function of the DNA, and which contain a phenotypic selection marker suitable for use in the identification of transformed cells. Markers, for example, are tetracycline resistance or ampicillin resistance. The word "vector" is sometimes used for cloning vehicle.

Expression vehicle. A vehicle similar to a cloning vehicle but which is capable of expressing a given structural gene in a host, normally under control of certain regulatory sequences.

Amplification. An increase in the copy number of a gene or plasmid.

Overexpression. Production of a protein at significantly higher levels than is present in a normal cell.

Copy number. The number of molecules, per genome, of a plasmid or gene which a cell contains.

Constitutive. An organism is said to be constitutive for the production of an enzyme or other protein if that protein is always produced by the cell under all physiological conditions.

Inducible. A gene or gene-product is said to be inducible if its transcription or synthesis is increased by exposure of the cells to an effector. For example, an inducible heat shock promoter is induced by the presence of high temperature.

Transformation or Transformed. A mechanism of gene transfer which involves the uptake of DNA by a host cell. Following entry into the cell, the transforming DNA may recombine with that of the host or may replicate independently as a plasmid.

Translation. The process of protein synthesis carried out by ribosomes which de-code the information contained in messenger RNA.

Transcription. The process of RNA synthesis upon a DNA template, mediated by RNA polymerase.

Detailed Description of the Invention

This invention provides for a method for high level production of a heterologous polypeptide or protein in a transformed host cell. The invention is based on the discovery that an inducible heat shock promoter operably linked to a heterologous gene, when amplified to a significant copy number, still retains a high level of inducibility. Using this method, increased production of the heterologous polypeptide or protein is achieved. Further, the method of this invention is particularly useful for the expression of heterologous polypeptides or proteins whose overexpression would impair growth of the host cell.

In the method of this invention, the heterologous gene encoding for the desired polypeptide or protein is under the control of an inducible heat shock promoter. The inducible heat shock promoter comprises one with no or low basal levels of transcription. Thus, prior to induction, this type of promoter will not express, or will express only very small quantities of, the polypeptide or protein under its control. Examples of inducible heat shock promoters with a low basal level of transcription include the Drosophila melanogaster 70,000 dalton heat shock protein gene promoter (hsp 70) described by Holmgren et al., Cell, 18: 1359-1370 (1979) and characterized in Amir et al., Molecular and Cellular Biology, 5:197-203 (1985) and in Corces et al., J. Biological Chemistry, 259:14812-14817 (1984). Heat shock promoters from a variety of eukaryotic organisms appear to be induced by a similar mechanism; indeed the sequence element responsible for induction is conserved from the fruitfly drosophila to humans (Pelham, Cell, 30: 517-528 (1982). Thus, any heat shock promoter isolated from a eukaryotic source, such as the drosophila hsp 22 and hsp 26 promoters (Ayme et al., J. Molecular Biology, 182: 469-475 (1985)), are examples of promoters that could be used in this system.

The inducible heat shock promoter can be obtained by isolating the heat shock gene from Drosophila, and then obtaining the promoter region enzymatically, chemically, or both. Similar promoters exist in every characterized eukaryotic species, and any of these species could be used as a source for the heat shock promoter. Further, the heat shock promoter may be synthesized de novo; for example, by manipulation in the laboratory rather than of natural origin.

In one embodiment of this invention, the heat shock promoter is operably linked to a structural gene and the resulting genetic construct is introduced into, or forms part of, an expression vehicle. The expression vehicle is then utilized to transform an appropriate host cell.

In another embodiment of this invention, the heat shock promoter is operably linked to a genetic sequence coding for a first polypeptide, and this genetic sequence is operably linked to a second genetic sequence coding for another polypeptide. The expression yields a fusion or precursor protein comprising both the amino acid sequence of the second polypeptide and that of the desired first polypeptide, and containing a selective cleavage site between them adjacent to the desired amino acid sequence.

The cleavage site is preferable methionine, although this site may be any preferred site known in the art. The desired polypeptide should preferably lack internal cleavage sites corresponding to the actual selected cleavage site. Other known cleavage sites include asn-gly, asp-pro, lys, arg, and lys-arg.

Selective cleavage of the fusion or precursor protein is typically effected outside of the replicative environment in the expression vehicle. In this post-translational step, the fusion or precursor protein is clipped by selective treatment. For example, when methionine is the cleavage site, the fusion or precursor protein is treated with cyanogen bromide to clip the desired polypeptide. With other known cleavage sites, the clipping treatment includes hydroxylamine, acid, trypsin, and lys-arg cleavage enzyme.

Methods for preparing fused, operably linked genes and expressing the same are known and are shown, for example, in U.S. patent 4,366,246.

Typically the desired structural genetic sequence is heterologous to the host cells, that is, it is not naturally produced by that host cell. Alternatively, the desired structural genetic sequence may be produced by the host cells, but in small quantities. Therefore, by use of the present invention, the yield of the desired protein can be increased.

After the transformation of the host cell, the copy number of the heterologous gene is increased through a gene amplification system. Gene amplification systems which can be used in the method of this invention are well known in the art, and may include dihydrofolate reductase (DHFR) amplification systems as described in U.S. Patent No. 4,399,216 to Axel et al. (columns 26-30). Other known methods of producing high copy numbers of a gene in cells include the adenoisine deaminase amplification system; or the use of vectors, such as SV40 or bovine papilloma virus (Mulligan, R. et al, Nature, 277: 108-114(1979); Lowy, D. P. et al, Nature, 287: 72-74 (1980), that allow autonomous replication in mammalian cells.

In the preferred method of this invention, the gene amplification system is the dihydrofolate reductase system (DHFR). This method involves transforming a DHFR-deficient host cell with an expresion vector containing the DHFR DNA. Transformed host cells with an altered phenotype, $DHFR^+$, can then be selected, and grown in the presence of increasing concentrations of methotrexate (MTX), which is an inhibitor of DHFR. Cells grown in increasing concentrations of MTX develop resistance to the drug by overproducing the DHFR enzyme, as a result of the amplification of the DHFR genes within the host cells' genome. When a cloning vehicle containing a heterologous gene of interest is cotransformed with the cloning vehicle containing the DHFR gene, it is known that both genes are amplified together under the selection pressure.

Thus, according to this invention, the gene amplification system is used to increase significantly the copy number of the heterologous gene operably linked to a heat shock inducible promoter. As will be understood by those skilled in the art, the copy number will vary from cell to cell and will depend upon the gene amplification system used. It is contemplated by this invention that the copy number of the heterologous gene will be significant, that is, more than about 100 copies per cell, more preferably greater than about 1,000 copies per cell.

After gene amplification, with the increased copy number, the heat shock promoter is induced by heat shock to the transformed host cells at a temperature and for a time sufficent to transcribe the heterologous gene. As is known in the art, the function of the promoter is totally defined by the cell type, for example, the drosophila hsp70 promoter is induced by different conditions in different cell types. Therefore, the heat shock temperature will depend upon the cell type used, but will typically be at $40^{\circ}C$ to about $45^{\circ}C$. The period of time for the heat shock will also depend upon the host cell type used, but will typically be about 15 minutes to about 180 minutes. During the heat shock, the heterologous gene is transcribed to messenger RNA, which is the template for the desired heterologous polypeptide or protein.

The heat shocked cells are then allowed to recover from the elevated temperatures of the heat shock. Typically the cells may be cultured at a lower temperature and for a time sufficient to translate the transcribed heterologous gene. By allowing the heat shocked host cells to recover from the heat shock, the induced transcribed heterologous gene (messenger RNA) is translated into high levels of the desired heterologous polypeptide or protein. The recovery temperature and time will depend upon the host cell used, but will typically be a recovery temperature of about $35^{\circ}C$ to about $39^{\circ}C$ and a recovery time of about 60 minutes to about 600 minutes.

The methods of this invention can preferably be used to express any polypeptide or protein. Examples of such polypeptides or proteins include, but are not limited to, enzymes, hormones, antibodies, structural proteins, interferons, interleukins, insulin, etc. The method of this invention can preferably be used to express structural gene products which impair growth of the host cells when they are overexpressed. Examples of such polypeptides or proteins include, but are not limited to, oncogenes, such as c-myc.

In the preferred embodiment of this invention, the heterologous gene is operably linked to the Drosophila heat shock protein 70 (hsp 70) promoter. An expression vector containing this genetic construct cotransforms a DHFR-deficient host cell, with an expression vector containing the DHFR gene under the control of a constitutive promoter. Constitutive promoters which may be operably linked to the DHFR gene include adenovirus late, EII, SV40 early and the like.

The genetic construct and methods involved herein can be utilized for expression of the polypeptides or proteins in mammalian host cells. The use of mammalian host cells are preferable when the desired structural gene to be expressed is a mammalian protein. The mammalian host cells are preferable with respect to glycosylation, phosphorylation, or other secondary modifications involved in mammalian protein systhesis. Examples of mammalian host cells include but are not limited to, Chinese hamster ovary cells (CHO), Namalva cells (Wurm et al., Devel. Biol Standard, 60: 393-404 (1985); BHK 21 cells (Radlett et al., Applied Mikrobiol., 22: 534-537 (19871); Mouse L cells (Hauser et al., Nature, 297: 650-654 (1982); and Vero Cells (Whittaker and Hayward, Develop. Biol. Standard, 60: 125-131 (1985).

In general, plasmid or viral (bacteriophage) vectors containing replicon and control sequences which are derived from species compatible with the host cell are used in connection with these hosts. These vectors ordinarily carry a replication site, and one or more unique restriction sites.

The cotransformed host can be fermented and cultured according to means known in the art to achieve optimal cell growth. Using the DHFR system, the host cells are grown in the presence of methotrexate. The concentrations of methotrexate are increased in a step-wise fashion, increasing the resistance of the host cells to methotrexate and providing for increased amplification of the gene. For example, initial cultures are carried in .005 uM methotrexate. Concentrations are increased in approximately four fold steps, and final concentrations can be as high as 1 mM.

The preferred fermentation procedure is as follows: the cotransformed host, preferably mammalian cells, more preferably Chinese hamster ovary (CHO) cells, is introduced into a culture medium containing nutrient materials that meet the growth requirements of the host cells. The host cells having the amplified heterologous gene are grown under culturing conditions selected to achieve maximum growth rate. Temperature conditions will depend upon the host, with the typical optimum range about 35°C to about 39°C, with 37°C being the most preferred for transformed CHO cells. The copy number of the heterologous gene will typically be about 100 to about 3,000.

After the cells have reached an optimal density, the heat shock promoter is induced. During this step, the temperature conditions are raised to about 40$^{O}$C to about 45$^{O}$C, with 43$^{O}$C being the most preferred temperature condition. The temperature is kept at this level for transcription of the heterologous gene into messenger RNA (mRNA), typically from about 15 minutes to about 180 minutes.

After the heat shock, the cells and mRNA are allowed to recover from the heat at a lower temperature and for a time sufficient to produce or translate the heterologous polypeptide from the messenger RNA template. The preferred temperature is about 35$^{O}$C to about 39$^{O}$C more preferably about 37$^{O}$C. Recovery and production time is typically from about 60 to about 600 minutes.

The produced heterologous polypeptide may be recovered according to means known in the art. Using the method of this invention, the level of polypeptide or protein produced is 20 to 100 fold higher than that produced by a constitutive promoter. The preferred embodiment of this invention is particularly useful when the heterologous polypeptide or protein impairs the growth of the transformed host cell. This method can accomplish induction of the promoter to expression of the heterologous gene within about 5 hours.

The following examples further describe the materials and methods used in carrying out the invention. The examples are not intended to limit the invention in any manner.

### Examples

In the following examples, these procedures were used:

## Cells

Dihydrofolate-reductase deficient CHO cells
(CHO-DUKX Bl) were obtained from L. Chasin, Columbia
University, New York. Urlaub, G. et al., Proc. Natl.
Acad. Sci. USA, 77:4216-4220 (1980). Prior to
transfection, cells were grown in alpha-modified MEM
with 10% dialized FCS to which adenosine, deoxya-
denosine and thymidine were added to a final concentration of 10 ug/ml each. Cells containing DHFR-
plasmids were maintained in alpha-modified MEM
supplemented with 10% dialyzed FCS and containing the
indicated amount of the drug methotrexate.

## DNA Transfection and Amplification

CHO-DUKX Bl cells were transfected by a modification of the calcium phosphate coprecipitation
technique of Graham and Van der Eb, described in
Graham, F.L. et al., Virology, 52:456-467(1973) and
Kingston, R.E. et al., Mol. Cell. Biol., 4: 1970-1977
(1984). Clones were picked using cloning cylinders
and six independent cell lines were established, from
which four cell lines (4-HS-MYC, 5A-HS-MYC, 5B-HS-MYC,
6A-HS-MYC) were grown in stepwise 4-fold increasing
concentrations of methotrexate, starting with a
concentration of 0.005 uM. Cells were allowed to
acclimate to each increased level of selection for two
to four weeks before again increasing the concentration of methotrexate. All cell lines were finally
established in 320 uM methotrexate.

## Plasmids

The plasmid pCVSVEII has been described in
Kingston, R.E. et al., Mol. Cell. Biol., 4: 1970-1977
(1984). The plasmid pHSmyc was constructed as follows: pSV2myc (Land, H. et al., Nature, 304:596-601 (1983)) was cut with Xba I, blunted with Klenow polymerase, then cut with BamHI in order to isolate the c-myc genomic fragment. This fragment was cloned into HincII and BamHI cut pSP6-HS-9 to obtain pHSmyc. pSP6-HS-9 was constructed by isolating the promoter containing HindIII/PstI fragment from p232.3 (Holmgren, R. et al., Cell, 18:1359-1370 (1979)), which contains a Drosophila heat shock protein 70 promoter, and then cloning this fragment into PstI/HindIII cleaved pSP65.

Analysis of RNA and DNA

Isolation and analysis of cytoplasmic RNA was performed as described in Favaloro, J. et al., Meth. Enzymology, 65:718-742 (1980) and Melton, D.A. et al., Nucl. Acids Res. 12:7035-7056 (1984) using radiolabelled RNA probes prepared with SP6 polymerase. Analysis of genomic DNA from cell lines was performed according to Southern using minigels for separation of restricted DNA and radiolabelled RNA-probes synthesized by SP6 polymerase.

Induction of Recombinant Cell Lines

Recombinant cells were split the day before induction to have a density of 60 to 80% of confluence in 10 cm petri dishes. They were heat shocked by feeding with medium that had been prewarmed to 43°C, and kept at that temperature in an incubator for 1 to 4 hours, preferably 2 hours. The medium was changed again after that period in order to have the cells recover at 37°C for 1 to 9 hours, preferably for 2 to 4 hours.

Analysis and Identification of c-myc Proteins

For lysis of cells 2 ml RIPA buffer (Klemens, R., Embo J., 4:2053-2060 (1985)) was added to 50-60% confluent cells in 10 cm dishes. Cells were scraped off immediately with the help of a rubber policeman and the highly viscous solution was sonicated 3 times 5 seconds each on ice. SDS-PAGE and Western blot analysis were performed using 12% gels (Hann, S.R., and Eisenman, R.N. (1984) Mol. Cell. Biol., 4:2486-2497 (1984)). For immunodetection of c-myc proteins polyclonal and monoclonal antibodies, raised against human c-myc protein, were used. These antibodies were a gift from R. Chizzonite, HofmannLaRoche, Nutley. To immunoprecipitate 32-phosphate labelled cellular proteins, 100 ul sonicated RIPA-buffer lysate was incubated with 25 ul protein A sepharose (Pharmacia) overnight at 4°C while rocking. The suspension was then centrifuged for 15 minutes at 13500 x g. One ul of monoclonal anti-c-myc antibody was added to the supernatant and the mixture was incubated at room temperature for 1.5 hour. Then 5 ul of rabbit anti-mouse IgG (affinity purified, 250 ug/ml, Cappel, Cooper Biomedical) together with 20 ul protein A sepharose was added. The suspension was incubated at 4°C for 2 hours on a rocking wheel and then centrifuged 10 minutes at 13500 x g. The sediment was washed 3 times with each 1 ml RIPA buffer containing 0.5 m NaCl before resuspending in 50 ul 2x Laemmli sample buffer. The sample was heated for 3 minutes at 100°C and an aliquot of 15 ul used for loading on the SDS-polyacrylamide gel. Radiolabelled S35-methionine and acid free Phosphate were obtained from NEN. Labelling of cellular proteins was performed by depleting the cells from the respective component during heatshock and adding the labelled component during the recovery time.

## Example 1

### Amplification of the c-ymc Gene in CHO Cells

An initial attempt was made to overexpress the c-myc protein by introducing the gene on a constitutive promoter (pSV2-cmyc) into the DHFR deficient CHO-DUKX Bl cell line using DHFR expression as a selection. Colonies that formed after this transfection had an abnormal phenotype; very few of the selected colonies grew into stable lines, and only one of the three resultant stable lines expressed c-myc RNA (data not shown). The cells of this line looked extremely refractile and grew poorly, suggesting that constitutive overexpression of c-myc may be cytotoxic. Therefore, plasmid pHS-myc was constructed. (Figure la). This plasmid links a Drosophila hsp70 promoter region, which has an extremely low basal level of expression, and the second and third exons of a mouse c-myc gene isolated from a plasmacytoma (Shen-Ong, G.1. et al., Cell, 31:443-450 (1982)). In pHA-myc, the first AUG 3' of the hsp70 transcription initiation site is that of the myc protein.

Figure la shows the construction of the expression vector pHS-myc. This plasmid contains a genomic mouse c-myc fragment from a unique XbaI site 48 bp upstream of the translation start codon in exon 2 to the unique BamHI site about 2.5 kb downstream of exon 3. The filled boxes denote the myc coding region, the hatched boxes denote the remainder of mouse c-myc genomic sequences. The open box denotes the drosophila hsp70 promoter region, and the arrow denotes the start site of the transcription. The fusion between the promoter region and the c-myc gene is at +88 of the 5' untranslated region of the hsp70 promoter.

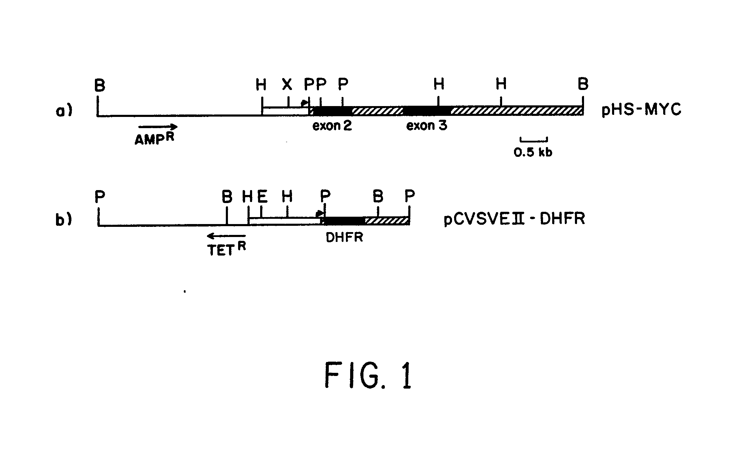

Figure 1b shows the DHFR selection vector pCVSVEII-DHFR. The DHFR coding region is denoted by the solid box; splice and polyadenylation signals are denoted by the hatched box; and the adenovirus EII promoter and SV40 enhancer region are contained in the area denoted by the open box. Details of this plasmid are found in Kingston, R.E. et al., Mol. Cell Biol., 4:1970-1977 (1984).

Plasmids pHS-myc (5 ug) and pCVSVEII-DHFR (1 ug) were introduced into DHFR deficient CHO DUKX B1 cells. Ten days after placing the cells in selection, individual colonies were cloned and subsequently expanded into cell lines. Six of the cell lines were tested for the presence of pHS-myc, and all contained the plasmid as determined by RNA analysis (described in Example 2). Four of these lines were cultivated in selective media containing stepwise increasing concentrations of the drug methotrexate, an antagonist of DHFR function. This selection pressure results in amplification of the transfected DHFR gene and associated DNA in order to allow cell viability (Alt, F.W. et al., J. Biol. Chem.,253:1357-1370 (1978) and United States Patent No. 4,399,216). After establishing these lines in 320 uM methotrexate, the copy number of the introduced c-myc gene in each cell line was determined and compared with the copy number of the introduced c-myc gene in a cell line growing at different levels of methotrexate.

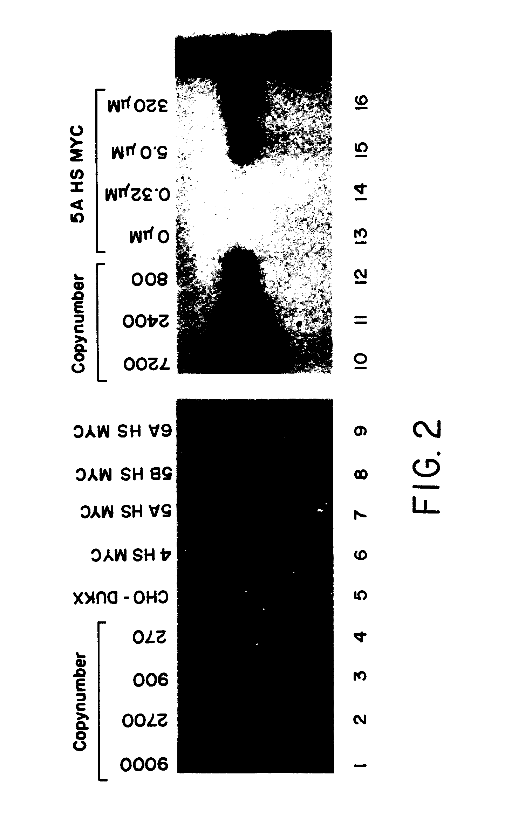

Figure 2 shows the copy number of the c-myc constructs in recombinant CHO cell lines. Genomic DNA was cleaved with HindIII and subjected to agarose gel electrophoresis and Southern transfer to nitrocellulose Genescreen Plus membranes (NEN). The membranes
were then hybridized to an RNA probe, SP6 polymerase
in vitro synthesized $^{32}$P-RNA containing the sequences
of the PstI-HindIII fragment of c-myc exon 2, intron 2
and exon 3. The last lane on the right contains
radiolabelled 1kb-ladder DNA (BRL). Lanes 6-9 show
genomic DNA (2 ug) isolated from four different recombinant cell lines growing at 320 uM methotrexate.
Lanes 13-16 show genomic DNA (0.5 ug) isolated from
the cell line 5A-HS-MYC growing at different levels of
methotrexate. Lanes 1-4 and 10-12 show genomic DNA
isolated from the plasmid pHS-myc.

In comparison to reconstructions made using the
plasmid pHS-myc, the recombinant cell lines contained
approximately 900 (cell line 4), 2700 (cell line 5A),
90 (cell line 5B) and 1500 (cell line 6A) copies of
the introduced c-myc gene. One of the four cell lines
(6A) contained additional rearranged c-myc genes as
well, as evidenced by the extra bands in Figure 2,
lane 9. These levels of DNA were the result of amplification of copy number during selection, as demonstrated for cell line 5A in Figure 2, lane 13-16.

## Example 2
### Induction of c-myc mRNA in Recombinant Lines

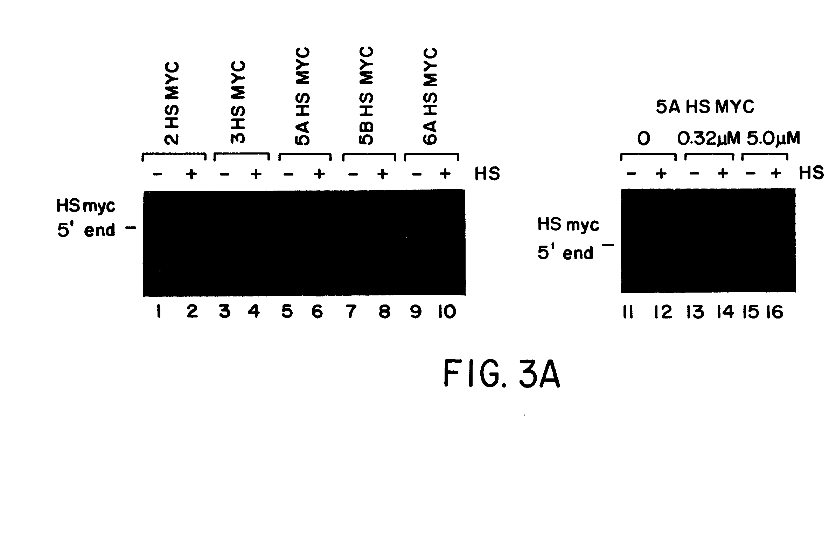

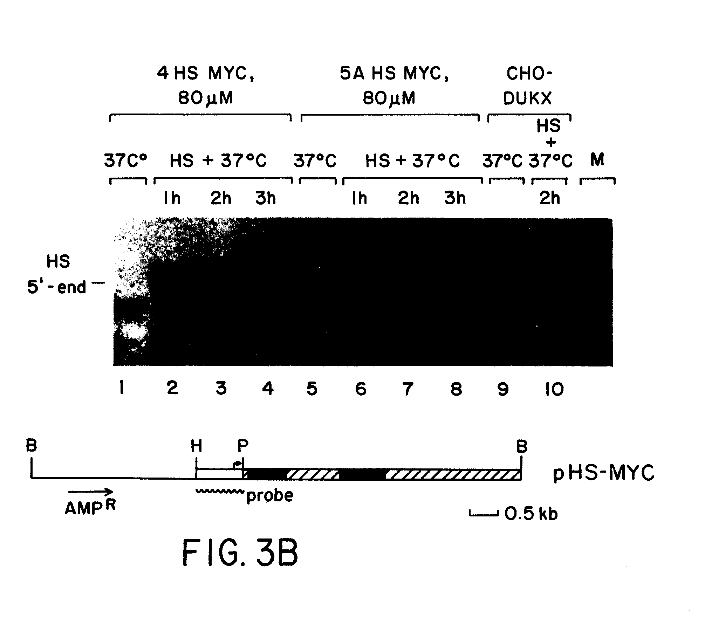

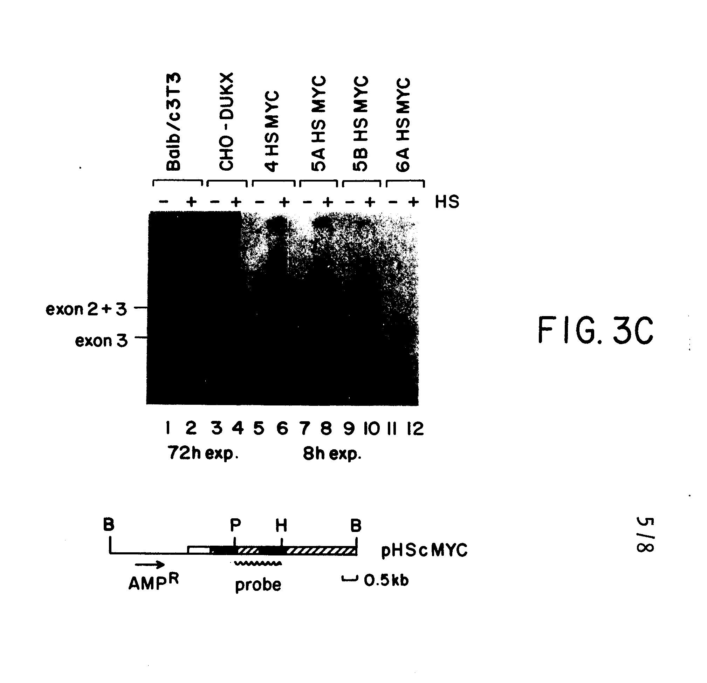

To determine whether transcription of the amplified c-myc genes in the recombinant cell lines could
be induced, RNA was isolated from growing cell lines
incubated for 2 hours at 37$^{\circ}$C or 43$^{\circ}$C. Figures 3, A
and B, show cytoplasmic RNA from uninduced (-) and
heat shock induced (+) (2 hours at 43$^{\circ}$C) recombinant

cells isolated and hybridized to $^{32}$P labelled RNA probe made using SP6 RNA polymerase that contained sequences of the entire Drosophila hsp70 promoter. The hybrid molecules were treated with RNaseI in order to cleave single stranded regions. The resulting molecules were separated on 6% non-denaturing polyacrylamide gels.

In Figure 3A, lanes 1-10 show the results of analysis of RNA samples (10 ug) from different recombinant cell lines as originally isolated in 0 methotrexate. The cell line used is indicated in the figure. Lanes 11-16 show the results of analysis of RNA samples (5ug) from the line 5A-HS-MYC growing at different levels of methotrexate. In both the original isolates of the cell lines (lanes 1-10) and the amplified cell lines (lanes 11-16), the amount of appropriately initiated message from the hsp70-c-myc fusion gene after heat shock was substantially increased. The level of RNA after induction increased as DNA copy number increased (lanes 11 to 16). The recombinant genes remain highly inducible even in the amplified cell lines (compare lanes 5 and 6 with lanes 15 and 16). It is difficult to quantitate the precise amount of induction due to the difficulty of detecting the basal level of expression.

Figure 3B shows RNA samples (3ug) from cell lines 4-HS-MYC (lanes 1-4), 5A-HS-MYC (lanes 5-8) and CHO-DUKX (lanes 9 and 10), analyzed as in A. RNA was isolated from cells growing at 37$^{\circ}$C (lanes 1, 5 and 9), or from cells that had been incubated at 43$^{\circ}$C for 2 hours and allowed to recover at 37$^{\circ}$C for 1 hour (lanes 2 and 6), 2 hours (lanes 3, 7 and 10) or 3 hours (lanes 4 and 8).

In order to determine the integrity of the message, an RNA probe covering the second and third exons of c-myc was used. This probe revealed the same primary RNA structure in the recombinant lines as for the endogenous c-myc gene in Balb-c 3T3 cells. Induced RNA levels in the recombinant lanes were at least 100 fold higher than observed for the endogenous gene of the Balb/c 3T3 cells. Figure 3C shows the comparison of c-myc mRNA sequences in Balb c/3T3, CHO-DUKX B1 cells and cells from recombinant CHO cell lines. Cytoplasmic RNA (5ug) from cells kept at 37$^{\circ}$C (-) and from cells induced for 2 hours at 43$^{\circ}$C (+) was isolated, hybridized to a radiolabelled SP6 polymerase created RNA containing sequences of the PstI-HindIII fragment of the mouse c-myc gene (exon 2, intron 2 and exon 3), and digested with RNaseI. The resulting molecules were analyzed on a 6% non-denaturing polyacrylamide gel. Lanes 1 and 2 (RNA from Balb/c 3T3 cells) and 3 and 4 (CHO DUKX cells) are shown as a 72 hour exposure, while lanes 5 to 12 (RNA from the indicated HS-MYC line) are an 8 hour exposure of the same gel.

A time course demonstrated that c-myc RNA levels continued to increase with increasing time of heat shock through 3 hours (data not shown). As c-myc protein synthesis does not occur in these lines at 43$^{\circ}$C (described in Example 3), it was of interest to determine if high RNA levels persisted when the heat induced cell lines were returned to 37$^{\circ}$C. RNA levels remained relatively constant for three hours following the temperature shift (Figure 3B).

## Example 3

### c-myc Protein Synthesis in Recombinant Lines

Initial attempts were made to detect the c-myc protein in recombinant cell lines immediately after a 2 hour heat shock at 43°C. There was no detectable c-myc protein made at that point as judged by pulse labelling with $^{35}$S-methionine or by immunoblotting. In contrast, however, when cells were allowed to recover at 37°C after the 2 hour heat shock, c-myc protein was detectable by immunoblotting and the level of protein increased with increasing time of recovery.

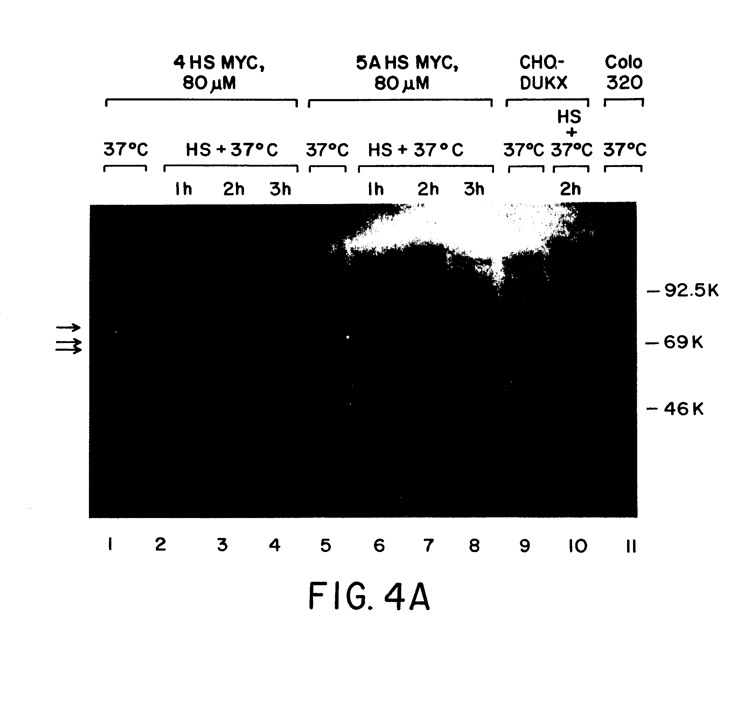

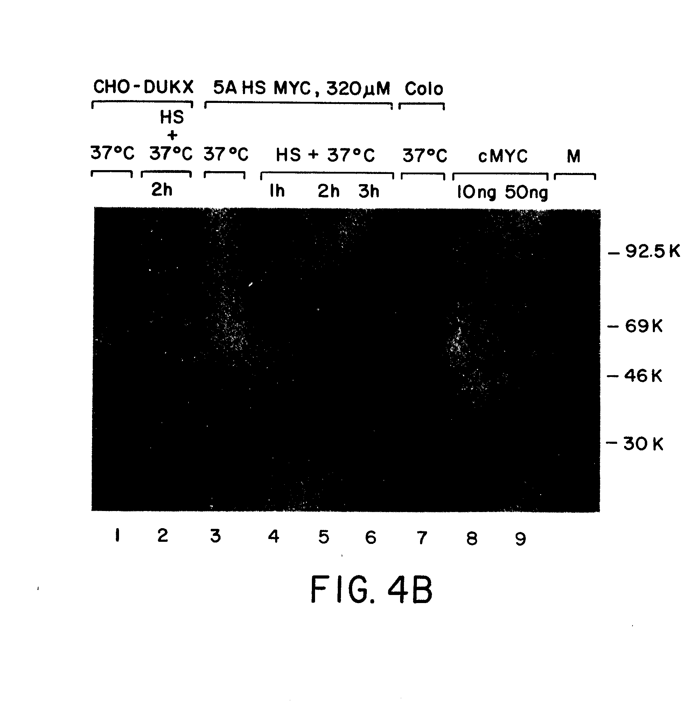

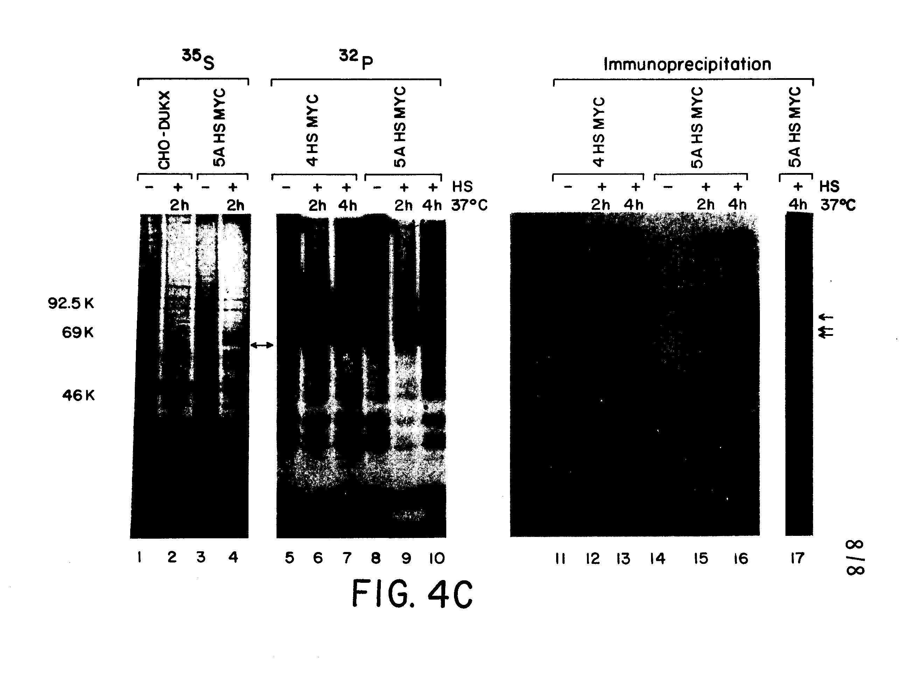

Figures 4, A and B, show the time course of induction of c-myc protein and analyses of protein levels in induced cells. RIPA-buffer lysates (2ml) were prepared from 70-80% confluent cells in 10 cm dishes after growing them at 37°C (Figure 4A: lanes 1, 5, 9, 11; Figure 4B: lanes 1, 3, 7) and after heat shock induction for 2 hours at 43°C and varying time period of recovery at 37°C (Figure 4A: lanes 2 and 6, 1 hour recovery; lanes 3, 7 and 10, 2 hours recovery; lanes 4 and 8, 3 hours recovery; Figure 4B: lane 4, 1 hour recovery; lanes 2 and 5, 2 hours recovery; lane 6, 3 hours recovery). Lysates were applied to 12% SDS-PAGE (20 ul per sample) and proteins transferred electrophoretically onto nitrocellulose filter paper (S+S, BA 85). c-myc proteins were detected by incubation with anti c-myc monoclonal antibodies (R. Chizzonite, Hoffmann-LaRoche; 1:2000 diluted) and $^{125}$Iodine labelled Protein A (NEN). In Figure 4B, lanes 8 and 9 contain purified c-myc (lane 8; 10 ug, lane 9; 50 ug) derived from recombinant insect cells (G. Ju, Hoffmann-LaRoche).

Three bands were observed with relative mobilities of 64,000, 66,000 and 75,000 that were recognized by either monoclonal or polyclonal antibodies raised against a portion of the human c-myc protein that is conserved in mouse c-myc (Miyamoto, C. et al., Mol. Cell. Biol., 5:2860-2865 (1985)). The smaller two of these have been seen in previous studies (Schwab, M. et al., Nature, 315:345-347 (1985); Hann, S.R. et al., Cell, 34:789-798 (1983); Ramsay, G., Proc. Nat'l. Acad. Sci. USA, 81:7742-7746 (1984); and Hann, S.R., and Eisenman, R.N. Mol. Cell. Biol., 4:2486-2497 (1984)).

The amount of c-myc protein produced by the 5A-HS-MYC line is at least twofold higher than the amount obtained from the 4-HS-MYC line, which has a lower copynumber of the c-myc construct. By comparing levels of protein with a purified protein produced in an insect expression vector (Miyamoto, C. et al., Mol. Cell. Biol., 5:2860-2865 (1985)), it was estimated that $10^9$ CHO cells produce approximately 1 mg of c-myc protein (Figure 4B, compare lanes 6 and 9). These lines produce substantially more c-myc protein than the human tumor line COLO 320 HSR (Schwabb, M. et al., Nature, 315:345-347 (1985)), which contains approximately 20 copies of an endogenous c-myc gene (compare lanes 8 and 11 in Figure 4A and lanes 6 and 7 in Figure 4B).

The presence of high levels of c-myc protein during the recovery period implies that c-myc is one of the more abundant proteins made in the recombinant lines during this time. This was verified as shown in Figure 4C. Figure 4C shows that the c-myc protein is one of the major proteins synthesized in the recombinant lines during recovery and it made as a phosphoprotein. RIPA buffer lysates of $^{35}$S methionine (lanes 1-14) and $^{35}$phosphate labelled proteins (lanes 5-10, immunoprecipitation: lanes 11-17), separated on 12% SDS-PAGE. Immunoprecipitation was performed as described above. Protein isolates were from the indicated cell line either growing at 37°C (-) or induced for 2 hours at 43°C (+) and allowed to recover at 37°C for either 2 hours (lanes 2, 4, 6, 9, 12 and 15) or 4 hours (lanes 7, 10, 13, 16 and 17). Lane 17 is a light exposure of lane 16. Analysis of the resultant cell lysates reveals that c-myc is one of the most actively synthesized proteins in cell line 5A during this period between 1 and 3 hours after heat shock (Figure 4C, lane 4).

The mammalian c-myc protein has been shown to be phosphorylated (Hann, S.R. et al., Cell, 34:789-798 (1983), Ramsay, G., Proc. Nat'l Acad. Sci USA, 81: 7742-7746 (1984), and Hann, S.R., and Eisenman, RN., Mol. Cell. Biol., 4:2486-2497 (1984)). The c-myc protein produced in recombinant cell lines 4 and 5A is also phosphorylated, and is the major phosphoprotein labelled during the recovery priod (Figure 4C, lanes 6, 7 and 9, 10). Immunoprecipitation reveals that all three species of the c-myc protein appear to be phosphorylated. (Figure 4C, lanes 12, 13, and 15-17). A culture of the 5A-HS-MYC cell line was deposited in American Type Culture Collection, 12301 Parklawn Dr., Rockville, Maryland 20852, under ATCC No. CRL 9010 on February 4, 1986. This depository assures permanence of the deposit and ready accessibility thereto by the public.

## Example 4
### Viability of Cell Lines After Induction of c-myc

Initial experiments had raised the possibility that overproduction of c-myc is cytotoxic. To test this hypothesis, the viability of the recombinant lines after induction of c-myc was investigated. Recombinant lines were plated at a density of approximately $10^6$/10cm dish, left at $37^O$C for 5 hours, and then either heat shocked for 2 hours at $43^O$C and then returned to $37^O$C, or left at $37^O$C. After 2 days at $37^O$C, no living cells were left on the dish of recombinant cells that had been heat shocked, while the control dish of the recombinant line as well as both the heat shock and control dishes of the parallel line were confluent. Similar results were obtained with the other three recombinant lines. Microscopic examinations of the cells during this recovery period revealed that they slowly died without further cell division after induction of c-myc.

The foregoing invention has been described in some detail by illustration and example for purposes of clarity and understanding. It would be obvious that certain changes and modifications may be practiced within the scope of the invention, as limited only by the scope of the appended claims.

Patent Claims:

1. A method for high level production of a polypeptide or protein in a transformed host cell comprising:

(a) transforming a host cell with a structural gene encoding for a polypeptide or protein, under the control of an inducible heat shock promoter;

(b) amplifying the copy number of said structural gene in said transformed host cells using an amplification system under the control of a promoter other than an inducible heat shock promoter;

(c) inducing said inducible promoter by heat shock to said transformed host cells at a temperature and for a time sufficient to transcribe said structural gene;

(d) allowing said heat shocked cells to recover at a lower temperature than said heat shock temperature and for a time sufficient to translate said transcribed structural gene, producing said polypeptide or protein.

2. The method of claim 1, wherein said amplification system of step (b) is selected from the group consisting of a dihydrofolate reductase amplification system, an adenosine deaminase amplification system, an SV40 vector and bovine papilloma virus vector.

3. The method of claim 1, wherein said heat shock promoter is an eukaryotic heat inducible promoter.

4.    The method of claim 3, wherein said heat shock promoter is selected from the Drosophila heat shock protein 70 promoter, the Drosophila heat shock protein 22 promoter, the Drosophila heat shock protein 26 promoter, and the human heat shock protein 70 promoter.

5.    The method of claim 1, wherein, after amplification, said copy number of said structural gene is at least about 100 copies per host cell genome.

6.    The method of claim 1, wherein said structural gene is heterologous to said host cell.

7.    The method of claim 1, wherein said structural gene is homologous to said host cell.

8.    The method of claim 1, wherein said host cell is a mammalian cell.

9.    The method of claim 8, wherein said mammalian host cell is a cultured mammalian cell selected from the group consisting of Chinese hamster ovary cells, Vero cells, Namalva cells, BHK21 cells, and mouse L cells.

10.  A method for increasing the production of a structural polypeptide or protein in a transformed mammalian cell comprising the steps of:

(a)  culturing a dihydrofolate reductase (DHFR) deficient mammalian cell transformed with

(i)  a DHFR gene under the control of a constitutive promoter, and

(ii) a structural gene encoding for a polypeptide or protein under the control of an inducible heat shock promoter,

in a culture medium containing methotrexate, for a time sufficient to permit growth of said transformed mammalian cells;

(b) inducing said heat shock promoter at a temperature and for a time sufficient to transcribe said structural gene; and

(c) allowing said heat shocked cells to recover from said heat shock at a lower temperature and for a time sufficient to translate said transcribed structural gene, producing said polypeptide or protein.

11. The method of claim 10, wherein said transformed mammalian cells are cultured in increasing concentrations of methotrexate.

12. The method of claim 10, wherein said heat shock promoter is induced at a temperature of from about 40°C to about 45°C and for a time of about 15 minutes to about 180 minutes.

13. The method of claim 10, wherein said recovery temperature of step (c) is from about 35°C to about 39°C and for a time of about 60 minutes to about 600 minutes.

14. The method of claim 10, wherein said mammalian cells are selected from the group consisting of Chinese hamster ovary cells, Vero cells, Namalva cells, BHK21 cells, and mouse L cells.

15. The method of claim 10, wherein said heat shock promoter is a Drosophila heat shock protein 70 promoter.

16. The method of claim 1 or 10 wherein said polypeptide or protein is selected from the group consisting of enzymes, hormones, antibodies, structural proteins, interferons, interleukins, insulin, and oncogenes.

17. A host cell cotransformed with:

(a) a gene amplification system under the control of a constitutive promoter, and

(b) a structural gene encoding for a polypeptide or protein under control of an inducible heat shock promoter.

18. The host cell of claim 17 wherein said gene amplification is selected from the group consisting of dihydrofolate reductase amplification system, an adenosine deaminase amiplification system, an SV40 vector, and bovine papilloma virus vector.

19. The cell of claim 17 or 18 wherein said inducible heat shock promoter is selected from the Drosophila heat shock protein 70 promoter, the Drosophila heat shock protein 26 promoter, the Drosophila heat shock protein 22 promoter, and human heat shock protein 70 promoter.

20. The cell of claim 17 or 18 wherein said polypeptide or protein is heterologous to said host cell.

0232845

1/8

FIG. 1

FIG.2

FIG. 3A

FIG. 3B

FIG. 3C

FIG. 4A

0232845

FIG. 4B

FIG. 4C

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

**Accession numbers of the deposits:**

ATCC No. CRL 9010